# EUROPEAN PATENT SPECIFICATION

(11) **EP 2 007 294 B1**
(45) Date of publication and mention of the grant of the patent: **22.03.2017**
(21) Application number: 07734232.7
(22) Date of filing: 06.04.2007
(51) Int. Cl.: A61B 17/54

(54) **DERMABRASION HANDPIECE**
DERMABRASIONS-HANDSTÜCK
PIÈCE À MAIN DE DERMABRASION

(30) Priority: 07.04.2006 IT BO20060264
(43) Date of publication of application: 31.12.2008
(73) Proprietor: GP Investimenti S.r.l., Montespertoli (FI) (IT)
(72) Inventor: NALDONI, Moreno, 50018 Scandicci (IT)
(74) Representative: Bergadano, Mirko
(86) International application number: PCT/IB2007/000913
(87) International publication number: WO 2007/116292

(56) References cited:
- FR-A- 2 765 471
- US-B1- 6 629 983

## Description

The present invention relates to a dermabrasion handpiece.

More specifically, the present invention relates to a handpiece adapted to perform micro-pulsed massages and to abrade the epidermis of a patient.

Skin abrasion handpieces are known in the field of cosmetics.

For example, a handpiece of this type is described US 6 629 983.

In a first embodiment described in this document, the distal end is provided with a central hole surrounded by an abrasive element. The hole is connected to a vacuum source for aspirating a portion of skin and for putting such portion of skin into contact with the abrasive element. The operator, by manually displacing the handpiece on the patient's skin, abrades the same.

In another embodiment described in the same document, a rotating blade is used to abrade the skin.

However, such handpieces only approximately solve the problem of performing a homogenous skin abrasion. Furthermore, some embodiments, such as that which contemplate a rotating blade, are potentially dangerous.

Therefore, it is the object of the present invention to make a dermabrasion device which is also capable of performing micro-massage actions on the patient's skin.

According to the present invention, a dermabrasion device is thus made according to the attached claims.

The present invention will now be described with reference to the accompanying drawings which illustrate a non-limitative example of embodiment thereof, in which:
- figure 1 shows an assembly view of the handpiece object of the present invention;
- figure 2 shows an exploded view of the assembly in figure 1;
- figure 3 shows a longitudinal section and two side views of a first element comprised in the handpiece shown in figures 1, 2;
- figure 4 shows a longitudinal section and two side views of a second element comprised in the handpiece shown in figures 1, 2;
- figure 5 shows a longitudinal section, a side view and two transversal sections of a third element comprised in the handpiece shown in figures 1, 2;
- figure 6 shows a side view of a fourth element comprised in the handpiece shown in figure 1, 2;
- figure 7 shows a side view, a longitudinal section and a plan view of a fifth element comprised in the handpiece shown in figures 1, 2; and

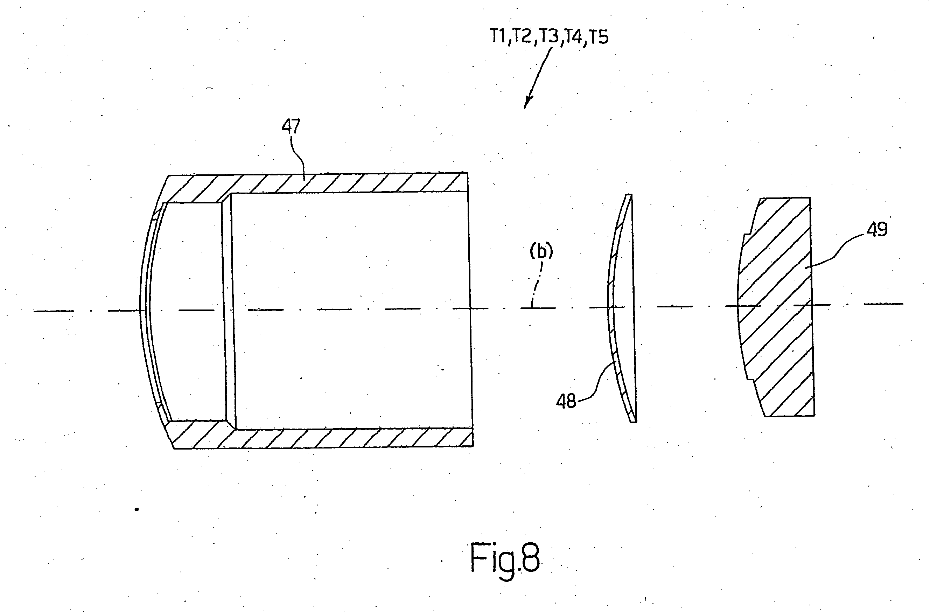

- figure 8 shows a section view of the components of a head used in the handpiece in figures 1 and 2.

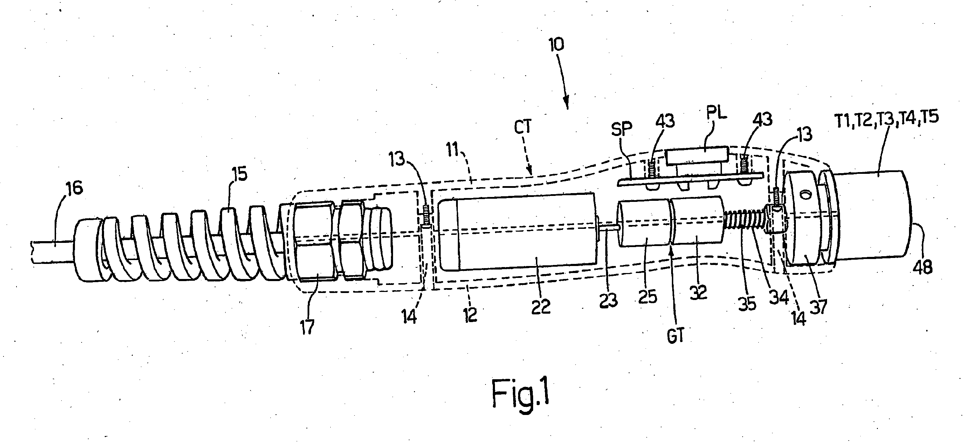

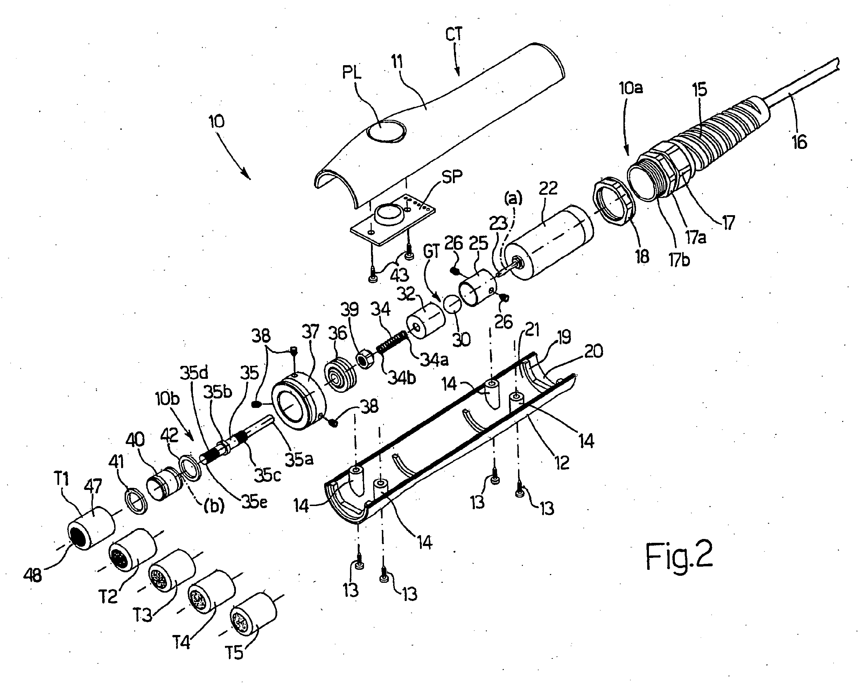

In figures 1 and 2, numeral 10 generically indicates, as a whole, an innovative handpiece adapted to abrade a user's skin and adapted to further perform micro-pulsed massages.

Handpiece 10 comprises a first half-shell 11 and a second half-shell 12, which, by means of four screws 13 (accommodated in corresponding seats 14) define a casing CT, which encloses therein a plurality of functional elements which will be described hereafter.

Proceeding from right to left in the exploded view in figure 2, handpiece 10 comprises on a first proximal end 10a a plastic spiral-like element 15 crossed by a flexible electrical wire 16 by means of which an electrical connection of handpiece 10 itself to a programmable dermabrasion machine (not shown) is obtained.

One end of electrical wire 16 end with a cable gland element 17 (integral with element 15), in turn fixed to half-shells 11, 12, by means of a washer 18 which is located on the other side with respect to an abutment 19 integral with second half-shell 12.

In other words, one portion 17a of cable gland element 17 rests on a surface 20 of second half-shell 12, while washer 18 is in contact with a surface 21, again of second half-shell 12. Washer 18 is screwed onto a threaded portion 17b of cable gland element 17, thus fixing cable gland element 17 to the two half-shells 11, 12.

Electrical wire 16 supplies a direct-current, low-voltage electrical motor 22, accommodated in use between two half-shells 11, 12.

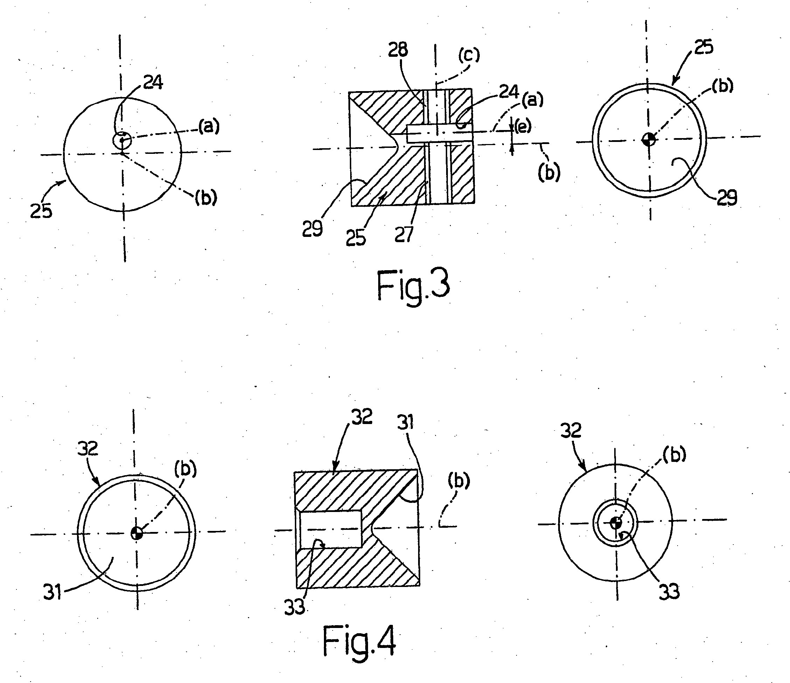

Electrical motor 22 presents a shaft 23 which is inserted in use in a hole 24 obtained in a first support 25 (figure 3).

As shown in figure 3, axis (a) of shaft 23 presents an eccentricity (e) with respect to a central axis (b) of handpiece 10.

Furthermore, shaft 23 is fixed to first support 25 by means of two threaded dowels 26, each of which is inserted in a corresponding threaded through hole 27, 28, with axis (c) perpendicular to axes (a) and (b). The free ends of threaded dowels 27 are tightened about shaft 23 and make it integral with first support 25 (figure 3).

As shown again in figure 3, first support 25 has a seat 29 (essentially conical) adapted to accommodate a first portion of a ball 30.

A second portion of ball 30 is contained in a seat 31 (also essentially conical) made in a second support 32 (figure 4) which is located in use on the opposite side of first support 25 with respect to ball 30.

The whole of first support 25, second support 32 and ball 30 creates a motion transmission joint GT having particular features, as better described below.

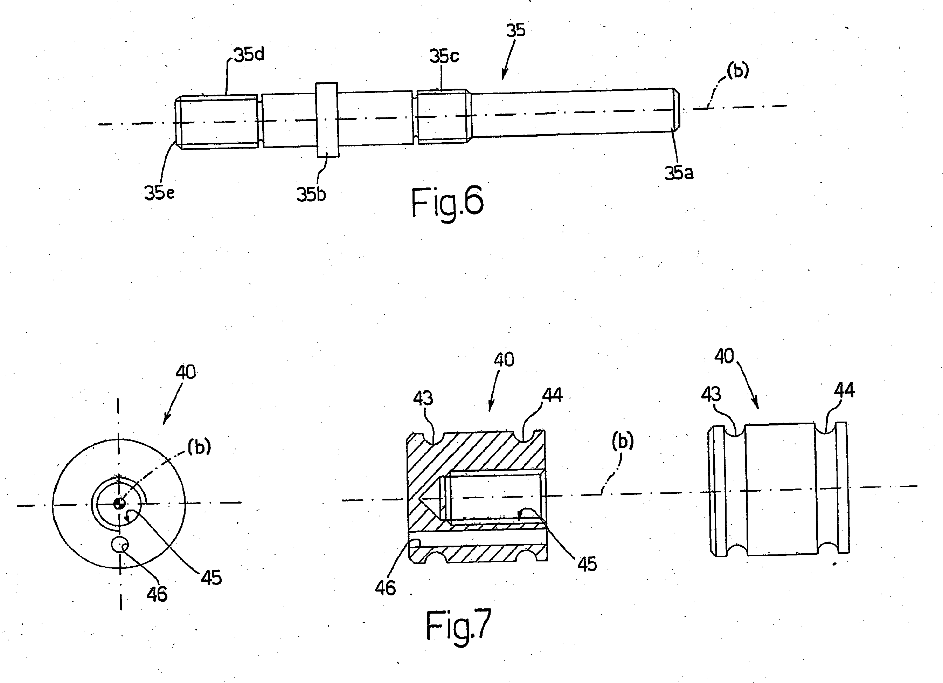

A seat 33 obtained in second support 32 accommodates a first end 34a of a spring 34 crossed by an end 35a of a head-holding shaft 35 (figure 6).

During assembly, end 35a of head-holding shaft 35 is further made to cross through a ball joint 36 (figure 2).

Furthermore, head-holding shaft 35 presents a shoulder 35b on which a second end 34b of spring 34 rests (figures 2, 6).

By comparing the two figures 3 and 4, it is inferred that seats 29 and 31 are identical, conical, and both with axis of symmetry (b).

Furthermore, head-holding shaft 35, like spring 34, has axis (b).

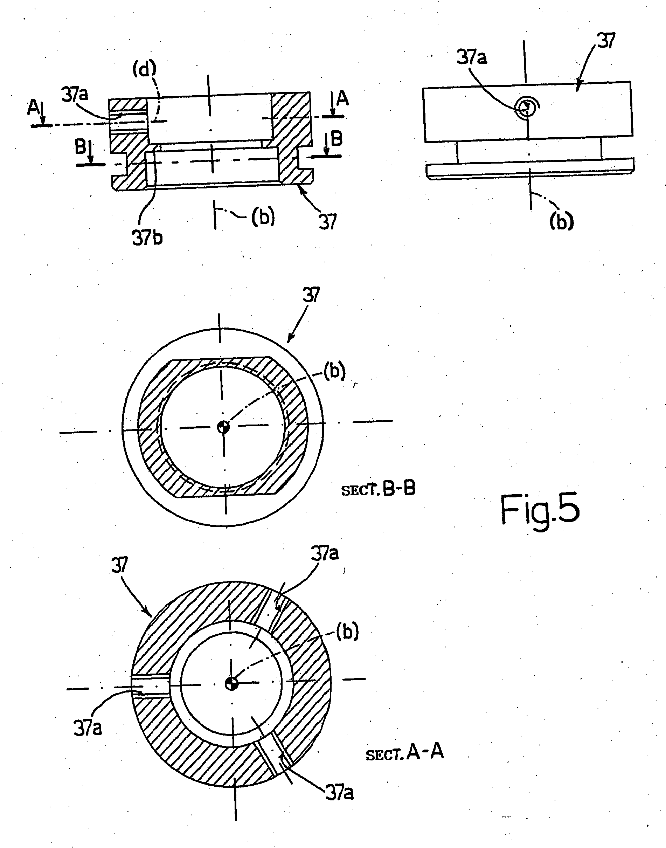

In turn, ball joint 36 is accommodated in a supporting bush 37 (figure 5). Furthermore, the external surface of ball joint 36 is locked onto support bearing 37 by the action of three dowels 38 arranged at 120° with respect to each other, each of which is fastened into a corresponding threaded seat 37a with axis (d) perpendicular to axis (b) (figures 2, 5).

The presence of such ball joint 36, in addition to the eccentricity (e) of shaft 23 with respect to axis (b) (figure 3), allows an "orbital" movement of head-holding shaft 35 and of the corresponding parts integral to it (see below), with positive effects on skin abrasion uniformity, precision and "smoothness".

Spring 34 has a two-fold function. In virtue of the intentionally present clearance, on one hand a certain beating, i.e. high-frequency vibration (micro-pulsation), of head-holding shaft 35 is allowed, and on the other hand ball 30 is maintained correctly in place between the two seats 29, 31, also in conditions of maximum effort or of creation of excessive clearance generated by the possible wear of seats 29, 31 due to the rolling action of ball 30 itself.

As shown in figures 2, 6, head-holding shaft 35 presents two threaded portions 35c, 35d.

A lock nut 39 (figure 2) is screwed onto threaded portion 35c to "sandwich" ball joint 36 against an abutment 37b provided in support bush 37 (figure 5).

A head-holding support 40, which allows to accommodate abrasive heads T1, T2, T3, T4, T5 by pressure (in virtue of the presence of two O-rings 41, 42) is fastened onto the other end 35e (presenting treaded portion 35d) of head-holding shaft 35.

Specifically, as shown in figure 7, head-holding support 40 presents an essentially cylindrical shape on whose outer surface two grooves 43, 44 are obtained, each of which is adapted to accommodate a corresponding 0-ring 41, 42.

Furthermore, a blank threaded hole 45 (with axis (b)) is obtained in head-holding support 40 into which end 35e of head-holding shaft 35 is fastened in use.

A cylindrical through hole 46, which allows the release of air from inside abrasive head T1, T2, T3, T4, T5 during its assembly on the head-holding support 40 extends parallelly to blank threaded hole 45.

Each head T1, T2, T3, T4, T5 comprises three parts.

Specifically, each head T1, T2, T3, T4, T5 comprises a cylindrical support 47 which is hinged onto head-holding support 40 (and kept still by the 0-rings 41, 42), a slightly rounded ring 48 of abrasive material (for example, silicon carbide, of variable grain among five different FEPA grain size values from 280 to 1200), and a plastic material plate element 49, which inserted in the plastic cylindrical support 47 allows to keep ring 48 itself in position.

Handpiece 10 is completed by an electronic board SP (fixed to the first half-shell by means of two screws 43) and an operating button PL of electrical motor 22 and thus of heads T1, T2, T3, T4, T5 (figures 1, 2).

In addition to heads T1, T2, T3, T4, T5 having variable grain size, shaft 23 of electrical motor 22 may be made to rotate at different speeds (e.g. at three different speeds) to implement different dermabrasion treatments.

All components 22, 23, 25, 26, 30, 32, 34, 39, 36, 37, 38, and a portion of 35 are contained within a space defined by the coupling of two half-shells 11, 12 by means of screws 13 (figures 1, 2).

The operation of handpiece 10 object of the present invention is easily inferred from description above.

The rotation of shaft 23 generates a similar rotation of first support 25. Motion is transmitted firstly to ball 30 and then to second support 32 to which shaft 35 is fixed. Since axis (a) of shaft 23 is offset by an eccentricity (e) with respect to axis (b) of the handpiece, an oscillation is generated on ball 30, oscillation which is transmitted to second support 32 and to shaft 35, which, being supported by ball joint 36, starts oscillating about axis (b).

The oscillations are thus transmitted to head-holding support 40 and to head T1, T2, T3, T4, T5 which is in use in that moment.

It must further be noted that the movement of ring 48 is not only vibratory but also oscillatory with respect to a laying plane initially perpendicular to axis (b). In this manner, an oscillatory motion of ring 47 with respect to a plane perpendicular to axis (b) is generated, which is transposed into a series of pleasant micro-massages on the patient's skin in addition to the dermabrasion action due to the vibratory motion about axis (b).

In use, an operator (not shown), after having carefully chosen one of heads T1, T2, T3, T4, T5 (according to the type of treatment to be performed), selects a rotation speed of shaft 23 (and thus the vibration speed ring 47 on the skin to be treated), for example by means of a sector situated on the dermabrasion machine (not shown) to which handpiece 10 is electrically connected. The operator then displaces ring 47 over the entire body surface to be treated.

The main advantage of above-described handpiece 10 object of the present invention consists in that by means of a simple mechanism comprising two supports 25, 32 and a ball 30 it is possible to transform the single rotational movement of shaft 23 of electrical motor 22 into vibrations and oscillations by means of which a pleasant massage is performed in addition to a dermabrasion on the patient's skin.

## Claims

1. A combined dermabrasion and skin massage handpiece (10); handpiece (10) comprising actuating means (22, 23), a head (T1, T2, T3, T4, T5) provided with at least one abrasive element (47) and a joint (GT) adapted to impress on the head (T1, T2, T3, T4, T5) a first oscillatory movement about a first axis (b);
wherein the joint (GT) further impresses on said abrasive element (47) a second oscillatory movement about a plane perpendicular to said first axis (b) to obtain skin micro-massages;
the handpiece (10) is **characterised in that** said joint (GT) comprises a ball (30) tightened between a first support (25) and a second support (32), said first support (25) being turned by an element (23) presenting a second axis (a) offset by an eccentricity (e) with respect to said first axis (b), said eccentricity (e) being adapted to produce an orbital movement of the head (T1, T2, T3, T4, T5) on the skin.

2. A handpiece (10) as claimed in claim 1, **characterised in that** said ball (30) is partially contained in a first seat (29) made in said first support (25), and partially in a second seat (31) made in said second support (32), which is aligned in use on the opposite side of said first support (25) with respect to said ball (30).

3. A handpiece (10) as claimed in claim 2, **characterised in that** said seats (29, 31) are essentially conical.

4. A handpiece (10) as claimed in claim 2 or claim 3, **characterised in that** said supports (25, 32) are pushed one against the other by elastic means (34).

5. A handpiece (10) as claimed in any of the preceding claims, **characterised in that** said second support (32) contemplates a seat (33) adapted to accommodate an end (35a) of a shaft (35).

6. A handpiece (10) as claimed in claim 5, **characterised in that** said shaft (35) is supported by a ball joint (36).

7. A handpiece (10) as claimed in claim 6, **characterised in that** said shaft (35) comprises means (35d) for fastening a head-holding support (40) adapted to support one of said heads (T1, T2, T3, T4, T5).

8. A handpiece (10) as claimed in claim 7, **characterised in that** said head-holding support (40) has at least two O-rings (41, 42) for rapidly fixing and releasing one of said heads (T1, T2, T3, T4, T5).

9. A handpiece (10) as claimed in any of the preceding claims, **characterised in that** the abrasive material with which said abrasive element (47) is made comprises silicon carbide, of variable grain among five different FEPA grain size values from 280 to 1200.

## Patentansprüche

1. Kombiniertes Dermabrasions- und Hautmassage-Handstück (10); wobei das Handstück (10) Betätigungsmittel (22, 23), einen Kopf (T1, T2, T3, T4, T5), der mit zumindest einem abrasiven Element (47) versehen ist, und ein Gelenk (GT) umfasst, das angepasst ist, eine erste Schwingbewegung um eine erste Achse (b) auf den Kopf (T1, T2, T3, T4, T5) aufzubringen;
wobei das Gelenk (GT) ferner auf das abrasive Element (47) eine zweite Schwingbewegung um eine Ebene senkrecht zu der ersten Achse (b) aufbringt, um Hautmikromassagen zu erzielen;
wobei das Handstück (10) **dadurch gekennzeichnet ist, dass** das Gelenk (GT) eine Kugel (30) umfasst, die zwischen einem ersten Träger (25) und einem zweiten Träger (32) befestigt ist, wobei der erste Träger (25) von einem Element (23) gedreht wird, das eine zweite Achse (a) versetzt um eine Exzentrizität (e) bezüglich der ersten Achse (b) darstellt, wobei die Exzentrizität (e) angepasst ist, eine Orbitalbewegung bzw. kreisförmige Bewegung des Kopfs (T1, T2, T3, T4, T5) auf der Haut zu erzeugen.

2. Handstück (10) nach Anspruch 1, **dadurch gekennzeichnet, dass** die Kugel (30) teilweise in einem ersten Sitz (29), der in dem ersten Träger (25) gemacht ist, und teilweise in einem zweiten Sitz (31) enthalten ist, der in dem zweiten Träger (32) gemacht ist, der bei Gebrauch auf der gegenüberliegenden bzw. entgegengesetzten Seite des ersten Trägers (25) bezüglich der Kugel (30) ausgerichtet ist.

3. Handstück (10) nach Anspruch 2, **dadurch gekennzeichnet, dass** die Sitze (29, 31) im Wesentlichen konisch sind.

4. Handstück (10) nach Anspruch 2 oder 3, **dadurch gekennzeichnet, dass** die Träger (25, 32) durch elastische Mittel (34) gegeneinander gedrückt werden.

5. Handstück (10) nach einem der vorhergehenden Ansprüche, **dadurch gekennzeichnet, dass** der zweite Träger (32) einen Sitz (33) vorsieht, der angepasst ist, ein Ende (35a) eines Schafts (35) aufzunehmen.

6. Handstück (10) nach Anspruch 5, **dadurch gekennzeichnet, dass** der Schaft (35) von einem Kugelgelenk (36) getragen ist.

7. Handstück (10) nach Anspruch 6, **dadurch gekennzeichnet, dass** der Schaft (35) Mittel (35d) zum Befestigen eines Kopfhalteträgers (40) umfasst, der angepasst ist, einen der Köpfe (T1, T2, T3, T4, T5) zu tragen.

8. Handstück (10) nach Anspruch 7, **dadurch gekennzeichnet, dass** der Kopfhalteträger (40) zumindest zwei O-Ringe (41, 42) zum schnellen Fixieren und Lösen eines der Köpfe (T1, T2, T3, T4, T5) aufweist.

9. Handstück (10) nach einem der vorhergehenden Ansprüche, **dadurch gekennzeichnet, dass** das abrasive Material, aus dem das abrasive Element (47) besteht, Siliziumkarbid umfasst, und zwar aus variablem Korn bzw. variabler Korngröße aus fünf unterschiedlichen FEPA-Korngrößenwerten von 280 bis 1200.

## Revendications

1. Pièce à main de dermabrasion et de massage de la peau combinée (10) ; la pièce à main (10) comprenant un moyen d'actionnement (22, 23), une tête (T1, T2, T3, T4, T5) dotée d'au moins un élément abrasif (47) et d'une articulation (GT) destinée à appliquer sur la tête (T1, T2, T3, T4, T5) un premier mouvement oscillatoire autour d'un premier axe (b) ;
dans laquelle l'articulation (GT) applique en outre sur ledit élément abrasif (47) un deuxième mouvement oscillatoire autour d'un plan perpendiculaire audit premier axe (b) pour obtenir des micro-massages de la peau ;
la pièce à main (10) est **caractérisée en ce que** ladite articulation (GT) comprend une bille (30) serrée entre un premier support (25) et un deuxième support (32), ledit premier support (25) étant tourné par un élément (23) présentant un deuxième axe (a) décalé par une excentricité (e) par rapport audit premier axe (b), ladite excentricité (e) étant destinée à produire un mouvement orbital de la tête (T1, T2, T3, T4, T5) sur la peau.

2. Pièce à main (10) selon la revendication 1, **caractérisée en ce que** ladite bille (30) est partiellement contenue dans un premier logement (29) réalisé dans ledit premier support (25), et partiellement dans un deuxième logement (31) réalisé dans ledit deuxième support (32), qui est aligné au cours de l'utilisation sur le côté opposé dudit premier support (25) par rapport à ladite bille (30).

3. Pièce à main (10) selon la revendication 2, **caractérisée en ce que** lesdits logements (29, 31) sont essentiellement coniques.

4. Pièce à main (10) selon la revendication 2 ou la revendication 3, **caractérisée en ce que** lesdits supports (25, 32) sont poussés l'un contre l'autre par un moyen élastique (34).

5. Pièce à main (10) selon l'une quelconque des revendications précédentes, **caractérisée en ce que** ledit deuxième support (32) comporte un logement (33) destiné à recevoir une extrémité (35a) d'un axe (35).

6. Pièce à main (10) selon la revendication 5, **caractérisée en ce que** ledit axe (35) est supporté par une liaison rotule (36).

7. Pièce à main (10) selon la revendication 6, **caractérisée en ce que** ledit axe (35) comprend un moyen (35d) destiné à fixer un support de maintien de tête (40) destiné à supporter une desdites têtes (T1, T2, T3, T4, T5).

8. Pièce à main (10) selon la revendication 7, **caractérisée en ce que** ledit support de maintien de tête (40) possède au moins deux joints toriques (41, 42) destinés à fixer et libérer rapidement une desdites têtes (T1, T2, T3, T4, T5).

9. Pièce à main (10) selon l'une quelconque des revendications précédentes, **caractérisée en ce que** le matériau abrasif avec lequel ledit élément abrasif (47) est réalisé comprend le carbure de silicium, de grain variable parmi cinq valeurs de tailles de grains FEPA différentes entre 280 et 1200.
